# EUROPEAN PATENT SPECIFICATION

(11) **EP 1 845 863 B1**
(45) Date of publication and mention of the grant of the patent: **08.06.2011**
(21) Application number: 06720182.2
(22) Date of filing: 02.02.2006
(51) Int. Cl.: A61B 17/00, A61B 17/12, A61F 2/06

(54) **POROUS MATERIALS FOR USE IN ANEURYSMS**
PORÖSE MATERIALIEN ZUR VERWENDUNG IN ANEURISMEN
MATERIAUX POREUX A UTILISER DANS DES ANEVRISMES

(30) Priority: 04.02.2005 US 51578
(43) Date of publication of application: 24.10.2007
(73) Proprietor: Stryker Corporation, Kalamazoo, MI 49002 (US); Stryker NV Operations Ltd, Dublin 2 (IE)
(72) Inventor: PORTER, Stephen, Christopher, Oakland, CA 94618 (US); CARR-BRENDEL, Victoria, E., Pleasanton, CA 94566 (US)
(74) Representative: Lecomte, Didier
(86) International application number: PCT/US2006/003751
(87) International publication number: WO 2006/084077

(56) References cited:
- US-A- 5 456 693
- US-A- 5 750 585
- US-A- 6 165 193
- US-A1- 2002 177 855
- US-B1- 6 245 090

## Description

Compositions and methods for repair of aneurysms are described. In particular, porous materials that enhance healing in the aneurysm are disclosed, as are methods of making and using these devices.

An aneurysm is a dilation of a blood vessel that poses a risk to health from the potential for rupture, clotting, or dissecting. Rupture of an aneurysm in the brain causes stroke, and rupture of an aneurysm in the abdomen causes shock. Cerebral aneurysms are usually detected in patients as the result of a seizure or hemorrhage and can result in significant morbidity or mortality.

There are a variety of materials and devices which have been used for treatment of aneurysms, including platinum and stainless steel microcoils, polyvinyl alcohol sponges (Ivalone), and other mechanical devices. For example, vaso-occlusion devices are surgical implements or implants that are placed within the vasculature of the human body, typically via a catheter, either to block the flow of blood through a vessel making up that portion of the vasculature through the formation of an embolus or to form such an embolus within an aneurysm stemming from the vessel. One widely used vaso-occlusive device is a helical wire coil having windings which may be dimensioned to engage the walls of the vessels. (*See, e.g.,* U.S. Patent No. 4,994,069 to Ritchart et al.) Other less stiff helically coiled devices have been described, as well as those involving woven braids. *See, e.g*., U.S. Patent No. 6,299,627.

U.S. Pat. No. 5,354,295 and its parent, U.S. Pat. No. 5,122,136, both to Guglielmi et al., describe an electrolytically detachable embolic device. Vaso-occlusive coils having little or no inherent secondary shape have also been described. For instance, co-owned U.S. Patent Numbers 5,690,666; 5,826,587; and 6,458,119 by Berenstein et al., describes coils having little or no shape after introduction into the vascular space. U.S. Patent No. 5,382,259 describes non-expanding braids covering a primary coil structure.

Vaso-occlusive devices comprising one or more coatings have also been described. U.S. Patent Nos. 6,280,457 discloses vaso-occlusive devices that include biodegradable coatings. U.S. Patent No. 6,602,261 describes vaso-occlusive devices comprising an elongate flexible carrier and hydrogel materials having a porosity less than 25 microns. U.S. Patent No. 6,245,090 describes vaso-occlusive devices comprising foam polymer materials having a porosity less than 250 microns with an open cell structure and including a radioopaque material. U.S. Patent No. 5,456,693 describes vaso-occlusive devices comprising a collagen plug having a porosity greater than 50 microns.
U.S. Patent No. 6,165,193 discloses a device for occluding a vascular site, such as an aneurysm, comprising a conformal vascular implant, formed of an expansible material, that is compressible from an initial configuration for insertion into the vascular site by means such as a microcatheter while the implant is in a compressed configuration, and that is expansible in situ into an expanded configuration in which it substantially fills the vascular site, thereby to embolize the site, wherein the initial configuration of the implant is a scaled-down model of the vascular site. U.S. Patent No. 6,165,193 fails to disclose an elastomeric porous material that has the claimed expansion behaviour and that has, in its fully expanded state, a nominal pore size greater than about 30 microns, wherein at least 50% of the pores are interconnected with an adjacent pore.

Thus, none of the above documents show implantable devices as described herein including one or more porous materials that limit the formation of scar tissue and resist long-term recanalization of an aneurysm.

The invention is defined by the features of the claims and relates to novel occlusive devices.

The porous material in any of the devices described herein may comprise one or more polymers, for example, silicones, polyterafluoroethylene, polyesters, polyurethanes, proteins, hydrogel materials and/or combinations thereof.

For the elastomeric material that is further expandable, pore size is preferably determined when the material is in the final expanded form. The elastomeric material comprises a porous material having a nominal pore size greater than about 30 microns in its fully expanded form, wherein at least 50 percent of the pores are interconnected with an adjacent pore. In certain embodiments, the pore size (in the fully expanded form) is between about 40 microns and about 400 microns. In other embodiments, at least 80% of the pores are interconnected with an adjacent pore.

In devices as described herein in which the porous material is expandable, the porous material may expand immediately upon deployment. Alternatively, in other embodiments, the porous, expandable material does not expand immediately upon deployment.

In another aspect, any of the vaso-occlusive devices described herein may further comprise one or more structural elements. In certain embodiments, the elastomeric, porous material at least partially surrounds the structural element(s). In other embodiments, the elastomeric material is at least partially surrounded by, the structural element(s). In still further embodiments, the porous material at least partially surrounds and is at least partially surrounded by the structural element(s). In any of the devices described herein comprising one or more structural elements, the structural elements may comprise a coil, a tubular braid, or combinations thereof.

In other aspects, any of the devices described herein may further comprise two or more additional members (*e.g*., structural elements such as coiled or braided member). In certain embodiments, the additional member(s) is itself a vaso-occlusive device. The porous material may surround and/or be surrounded by the additional structural element(s). In certain embodiments, the porous material is attached to the structural member(s) at one or more locations. In any of the devices described herein, the additional member(s) may comprise a metal (*e.g*., nickel, titanium, platinum, gold, tungsten, iridium and alloys or combinations thereof), stainless steel or a super-elastic metal alloy. The structural element(s) may be, for example, be shaped as a helical coil. In any of the devices described herein, the structural element(s) may further comprise a biodegradable material and/or a bioactive component.

Any of the devices described herein may further comprise a severable junction detachably which may be connected to a pusher element. The detachment junction can be positioned anywhere on the device, for example at one or both ends of the device. In certain embodiments, the severable junction(s) are, an electrolytically detachable assembly adapted to detach by imposition of a current; a mechanically detachable assembly adapted to detach by movement or pressure; a thermally detachable assembly adapted to detach by localized delivery of heat to the junction; a radiation detachable assembly adapted to detach by delivery of electromagnetic radiation to the junction or combinations thereof. The detachment junction(s) may be attached to porous material or one or more additional vaso-occlusive members.

A method of occluding a body cavity is described, the method comprising introducing any of the implantable devices as described herein into the body cavity. The body cavity may be an aneurysm.

These and other embodiments of the subject invention will readily occur to those of skill in the art in light of the disclosure herein.

### BRIEF DESCRIPTION OF THE FIGURES

FIG. 1 is a perspective view of an exemplary porous material as described herein having interconnected pores for use in promoting wound healing in an aneurysm, typically in combination with a structural element (*e.g*., vaso-occlusive coil).

FIG. 2 is a partial side-view, partial cross-section view of an exemplary device comprising an implantable porous material as described herein surrounding a coil-shaped vaso-occlusive device.

FIG. 3 is a partial side-view, partial cross-section view of an exemplary device according to FIG. 1. Within the deployment catheter, the porous material is shown in a compressed configuration.

FIG. 4 is a partial side-view, partial cross-section view of an exemplary device comprising an implantable porous material as described herein in combination with a tubular braided covering.

FIG. 5 is a partial side-view, partial cross-section view of an exemplary device comprising an implantable porous material as described herein in combination with a coil shaped outer covering.

FIGs. 6A to 6C are schematic side, overviews depicting an exemplary porous material that expands upon sufficient hydration. FIG. 6A depicts the material in its compressed form (*e.g*., for delivery through a catheter). FIG. 6B shows the material upon release from a catheter and FIG. 6C shows expansion (swelling) of the material upon exposure to water. The pores are sized to accommodate the swelling.

FIG. 7 is a side-view, partial cross-section view of an exemplary device comprising a porous material as described herein being deployed or retracted into a catheter. The material is easily retracted by compression of the pores (voids) as it is re-sheathed into the catheter.

It is to be understood that the drawing depicts only exemplary embodiments and are not to be considered limiting in scope.

Occlusive (*e.g*., embolic) compositions are described. The implantable porous biomaterials described herein have an appropriate architecture that promotes new vessel formation and maintains healthy viable tissue within and around the implant. Methods of making and using these vaso-occlusive elements are also described.

It must be noted that, as used in this specification and the appended claims, the singular forms "a", "an", and "the" include plural references unless the content clearly dictates otherwise. Thus, for example, reference to an implant comprising "a channel" includes implants comprising of two or more of such elements.

The implantable devices described herein comprise an elastomeric, compressible material that is made up predominately of void space (pores) biomaterial that is space-filling within an aneurysm and promotes long term, persistent foreign body responses to the material that does not become scar tissue. By "elastomeric" is meant a material that is capable of recovering size and shape after deformation (*e.g*., compression). Thus, the materials described herein can be substantial compressed, for example for delivery through the lumen of a catheter, and upon deployment, self-expand to the uncompressed volume.

The voids (pores) allow for significant compression of the material (*e.g*., in thickness) and the elastomeric nature allows the material to return to it non-compressed form when the pores are not compressed. Thus, an advantage of the porous materials described herein is that the air can be forced out of the pores (the material can be compressed) until the material is delivered, and then the material can relax back to its preferred (native) state. This offers the advantage of delivering the material through smaller diameter catheters than the final, non-compressed material.

Another advantage of the elastomeric nature of the porous materials described herein is that they can readily be retracted into the delivery mechanism (*e.g*., catheter) by compressing the pores. This allows the operator to easily retrieve and/or re-position the devices.

According to the invention, the porous materials described herein are capable of expanding (swelling) from the non-compressed state, for example upon exposure to a sufficient amount of water. The amount of hydration sufficient to cause expansion may be such that expansion is immediate upon deployment (contact with water). Alternatively, it may require time to reach the amount of hydration sufficient to cause expansion thereby delaying expansion from the uncompressed volume so that the physician can position the device as desired.

The time period during which expansion is delayed may be as short as 30 seconds or as long as 1 hour or even longer, including any time therebetween (*e.g*., 1 minute, 2 minutes, 3 minutes, 4 minutes, 5 minutes, 6 minutes, 7 minutes, 8 minutes, 9 minutes, 10 minutes, 15 minutes, 20 minutes, 25 minutes, 30 minutes, 35 minutes, 40 minutes, 45 minutes, 50 minutes, 55 minutes, 60 minutes or even longer). Hydrogel materials are known in the art and described herein. *See, also,* U.S. Patent Nos. 6,979,344; 6,960,617; 6,913,765; 6,855,743; 6,818,018; 6,676,971; 6,616,617; 6,463,317; 6,152,943; 6,113,629; 5,510,418; 5,328,955; and 5,258,042.

Furthermore, the time which it takes for the non-compressed material to reach its fully expanded state (*e.g*., via water diffusion) may be determined by any suitable means, including, but not limited to, selection of materials having an inherent hydrophobicity that results in the desired delay in swelling; selection of materials having inherent expansion characteristics that delay expansion (*e.g*., by requiring diffusion of ions from blood into the material and/or changes in pH, *see, e.g.,* U.S. Patent Publication Nos. 2005/0004660 and 2003/0014075); coating or otherwise sheathing (enclosing) some or all of the expandable material in a soluble material which delays water diffusion into the elastomeric, expandable material; coating or otherwise sheathing some or all of the expandable material with a insoluble material which acts as a water diffusion barrier into the underlying material; selection of materials that are highly lipophilic and swell in response to absorption of blood soluble lipids (*e.g*., since free lipids are in low concentration within the blood the rate of swelling would be slow) and/or partially or filling some or all of the pores with a soluble binder (*e.g*., one or more water soluble polymers and/or one or more water soluble proteins) which keeps the compressed material in a collapsed state until the binder has sufficiently dissolved.

The porous materials described herein expand to at least 200% (or any amount above 200%, including but not limited to, 250%, 300%, 350%, 400%, 450%, 500%, 600%, 700%, 800%, 1000% or even more) of its original uncompressed (native) volume. It will be apparent that when the material is expandable, the pores are sized appropriate to accommodate expansion (*e.g*., during hydration).

Thus, the elastomeric material described herein will have a "fully expanded state." For certain materials (*i.e*., materials that do not expand further from their non-compressed state), the fully expanded state is the non-compressed state. For materials that expand further from the non-compressed state, the fully expanded state occurs when the materials has obtained its fully expanded conformation, *i.e*., is both non-compressed and expanded from this non-compressed state.

In certain embodiments, the porous material is a macroporous material when in its fully expanded state. By "macroporous" is meant that a material that having a porosity of greater than 40 microns, generally between about 40 and about 400 microns (or any value therebetween), for example from about 40 to about 100 microns (or any value therebetween), from about 100 to about 200 microns (or any value therebetween), from about 200 microns to about 300 microns (or any value therebetween), or from about 300 to about 400 microns (or any value therebetween). Generally, the pores of the macroporous material are amorphous in shape.

Some or all of the pores of the vaso-occlusive porous materials described herein are interconnected. At least 50% of the pores are interconnected with an adjacent pore. In certain preferred embodiments, at least about 50% to about 80% (or any value therebetween) of the pores are interconnected.

By "biomaterial" is meant any substance or combination of substances synthetic or natural in origin, which can be used for any period of time, as a whole or as a part of a system that treats, augments, or replaces any tissue, organ, or function in a subject (*e.g*., mammal).

Aneurysms treated with known vaso-occlusive devices may result in the formation of scar tissue over time. Because scar tissue is an avascular, acellular mass made up mostly of extracellular matrix proteins, the interface between the scar tissue and healthy tissue is less robust than surrounding tissue and, as such, less resistant to long-term recanalization. The vaso-occlusive devices described herein comprise materials that are capable of arresting wound healing in a state of a modified foreign body response (also referred to as material microarchitecture-driven neovascularization) so that the resulting tissue response does not evolve into-scar and, as such, is adjacent (continuous) with the healthy tissue and is more resistant to sheer, flow and recanalization. Notably, there is no demarcation between the response to the foreign body and the native tissue - they are co-continuous.

During a typical course of wound healing, neutrophils are the predominant cell type at the site of injury within the first 24-48 hours, killing and phagocytosing bacteria and/or cellular debris. After approximately 48 hours, macrophages become the predominant cell type, further removing cellular and foreign debris from the wounded area. Within three to four days, fibroblasts migrate out of the surrounding connective tissue (*e.g*., intima) into the wound area and begin to synthesize collagen, which quickly fills the wound space, forming a complex tertiary structure consisting of both cells and extracellular matrix components. New blood vessels also begin to grow into the area at this time to supply oxygen and nutrients needed by the metabolically active fibroblasts and macrophages. However, in a typical course of wound healing, new vessel formation begins to regress in the second week, resulting formation of an avascular and acellular scar.

-Porous biomaterials as described herein provide tissue biomaterial anchoring and promote in-growth throughout the pores. The resulting "hallway" or "channel" pattern of tissue growth are healthy, space-filling masses that persist over the duration of the implant and promote host cell integration between the aneurysm wall and implant and throughout the aneurysm space.

For example, in embodiments in which most or all of the pores of the biomaterials described herein are preferably interconnected (co-continuous), the co-continuous pore structure of the biomaterials promotes space-filling in-growth of cells between aneurysm wall and implanted material. Thus, the porous implants described herein promote appropriate wound healing within the aneurysm environment and throughout the porous material. At least 50% of the pores have interconnections with adjacent pores. More preferably the inter connectivity is above at least about 80%.

Additionally, the porous materials described herein having interconnected pores are mostly void space, consisting of a lattice work that cells grow between and around. Although other examples may exist where porous material is used within an aneurysm, the materials do not specifically call out the need for the host cells to in-grow and become co-continuous with other host cell-filled pores. *See, e.g*., International Patent Publications WO 04/078023; WO 04/103208; WO 04/062531; and WO 04/037318. Other technologies may allow for protein adsorption or absorption, but again do not promote cellular in-growth throughout via an interconnected porous structure. Thus, unlike other described porous biomaterials, the co-continuous pore structure of certain materials described herein promotes host cell in-growth with concomitant neovascularization, and, in addition, that enhances cell and vessel persistence within the pores.

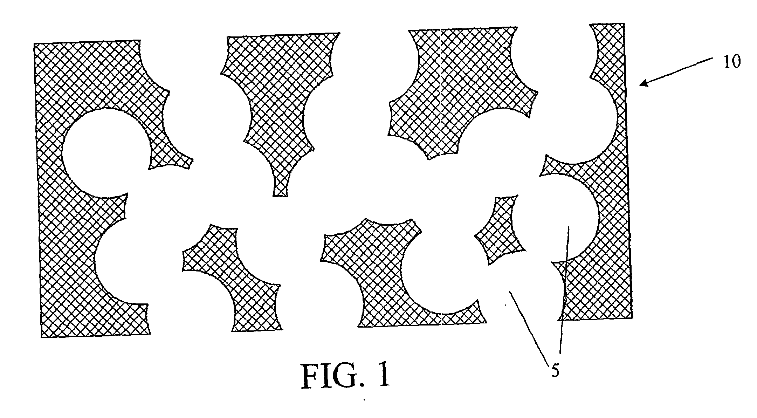

FIG. 1 depicts an exemplary embodiment of the inventive porous implants described herein. The device as a whole is generally designated (10) and is shown in a three-dimensional block. FIG. 1 shows an embodiment in which all of the pores (5) are interconnected.

The porous implants as described herein preferably comprise one or more materials that favor an arrested foreign body response, which as described above is granular in nature, has new vessel formation.

Non-limiting examples of suitable porous materials include natural and synthetic materials such as acrylates, silicones, ePTFE, urethanes (*e.g*., polyurethane), collagens and/or hydrogels. Copolymers of these materials which incorporate hydrophilic segments, such as polyethylene oxide, polyvinyl alcohol, polyacrylamide, polysaccharides, and other polymers known to form gels may also be used.

Methods for introducing suitable porosity in these materials are well known and include but are not limited to, addition of excipients during the formation of the material followed by removal of the excipient (*e.g*., by dissolving); use of a blowing agent or high pressure gas which rapidly expands during formation/extrusion or is permeated within the material structure at high pressure, followed by rapid decompression to form many microbubbles. *See, also,* U.S. Patent No. 4,076,656; U.S. Patent No. 5,681,572; U.S. Patent No. 6,602,261 as well as International Patent Publications WO 04/078023; WO 04/103208; WO 04/062531; and WO 04/037318.

Although the use of some of foam materials have been used as scaffolds to promote granular tissue in-growth, (*see*, *e.g*., U.S. Patent No. 6,713,079, Seare et al (1993) ASAIO Journal 39: M668-M674) has been described, these foams have not been used as vaso-occlusive devices, likely because of their known anti-thrombogenic characteristics or lack of delivery systems to the vasculature.

Furthermore, although hydrogel and other materials have been proposed for use in aneurysm repair (*see, e.g.,* U.S. Patent No. 6,818,018 and 6,602,261), these hydrogels have a porosity of less than 25 micron. Additionally, some hydrogels are incompatible with large pore architectures as gels may lack the strength to be deployed and maintain association between pores (*e.g*., they fracture or break apart). Furthermore, unlike previously described foam polymer devices (*see, e.g.,* U.S. Patent No. 6,245,090 and 5,456,693), the porous materials described herein expand (*e.g*., upon sufficient hydration, to a volume at least 200% of the uncompressed volume), wherein at least about 50% of the pores are interconnected. As described herein, interconnectedness between the pores may induce the type of persistent granular tissue that will result in durable aneurysm treatment.

The porous materials of the devices described herein may include one or more fibers, strands, coils, globules, cones or rods of amorphous or uniform geometry that are smooth or rough.

The porous devices described herein can also be optionally used in combination with other vaso-occlusive members, for example the GDC-type vaso-occlusive coils described above (*see, e.g.,* U.S. Patent Nos. 6,723,112; 6,663,607; 6,602,269; 6,544,163; 6,287,318; 6,280,457 and 5,749,894).

As noted above, the porous materials may be macroporous in that they comprise one or more materials having an average pore size ranging from approximately 20 microns to about 400 microns (or any value therebetween), more preferably from about 30 microns to about 300 microns (or any value therebetween), and even more preferably from 40 microns to about 200 microns (or any value therebetween), using conventional methods for determination of pore size (porosity) in the trade.

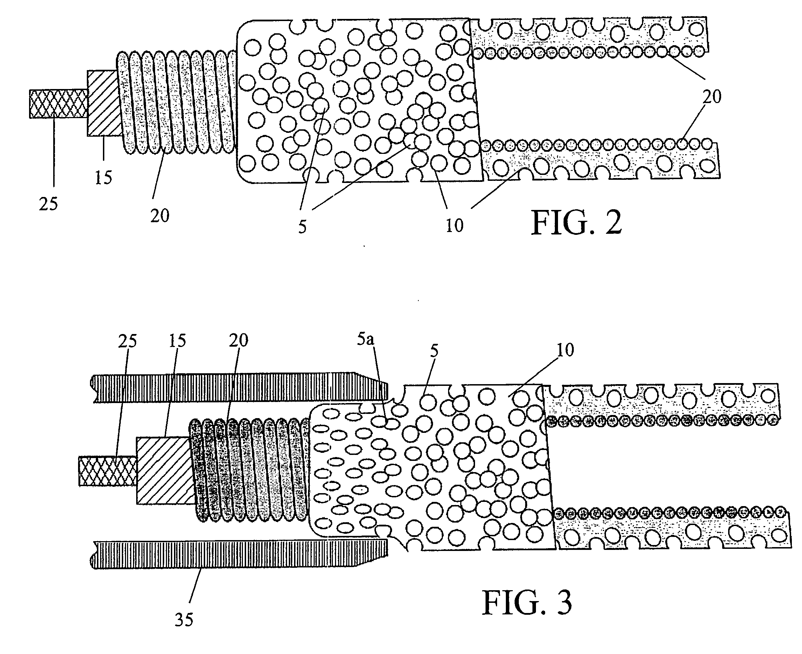

FIG. 2 shows an exemplary embodiment depicting a porous material as described herein in combination with GDC-type vaso-occlusive coil (20). As shown in FIG. 2, the porous material may have a tubular shape that surrounds an inner vaso-occlusive member. Porous material may also extend into part or all of the lumen of the coil (20). Interconnected (co-continuous) pores (5) are depicted as overlapping circles. The porous component (10) can be permanently or temporarily attached in one or more locations to the coil (20) by any suitable attachment mechanism. Also shown is detachment junction (15) positioned on the proximal end of the coil (20) as well as pusher wire (25).

As noted above, the porous devices, described herein are compressible, for example for loading into a deployment catheter. FIG. 3 shows the exemplary embodiment of FIG. 2 as partially deployed from a deployment catheter (35). Within the catheter (35), the pores (5a) of macroporous component (10) are compressed. Upon deployment, the pores (5) expand to their relaxed state.

The devices described herein may also include one or more outer members covering the porous member. Thus, the porous member may surround and/or be surrounded by one or more structural members.

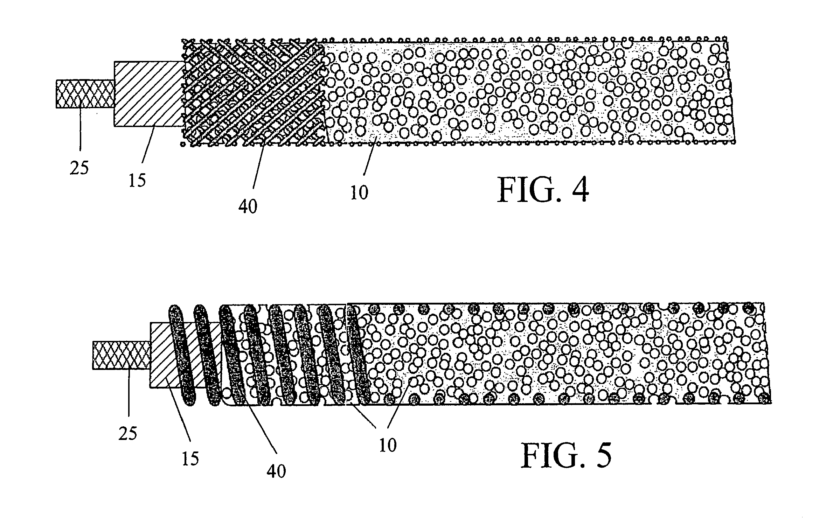

FIG. 4 shows another exemplary embodiment in which the porous component (10) is surrounded by an outer component (40). In this embodiment, outer component (40) comprises a tubular braid.

FIG. 5 shows another exemplary embodiment in which the porous component (10) is surrounded by an outer component (40), the outer component (40) having a coil shape in this embodiment.

The optional additional members (inner or outer) may assume a variety of structures. Thus, in addition to the coils and braids depicted in the Figures, other shapes are contemplated including, but not limited to, wires, knits, woven structures, tubes (*e.g*., perforated or slotted tubes), injection-molded devices and the like. *See, e.g.,* U.S. Patent No. 6,533.801 and International Patent Publication WO 02/096273.

Additionally, the additional structural member(s) (*e.g*., vaso-occlusive members) may be made of a variety of materials, including but not limited to metals, polymers and combinations thereof. In certain embodiments, the additional member(s) (*e.g*., braid, coil, etc.) comprises one or more metals or metal alloys, for example, Platinum Group metals, especially platinum, rhodium, palladium, rhenium, as well as tungsten, gold, silver, tantalum, stainless steel and alloys of these metals. Preferably, these elements comprise(s) a material that maintains its shape despite being subjected to high stress, for example, "super-elastic alloys" such as nickel/titanium alloys (48-58 atomic % nickel and optionally containing modest amounts of iron); copper/zinc alloys (38-42 weight % zinc); copper/zinc alloys containing 1-10 weight % of beryllium, silicon, tin, aluminum, or gallium; or nickel/aluminum alloys (36-38 atomic % aluminum). Particularly preferred are the alloys described in U.S. Pat. Nos. 3,174,851; 3,351,463; and 3,753,700. Especially preferred is the titanium/nickel alloy known as "nitinol." A shape memory polymer such as those described in International Publication WO 03/51444 may also be employed.

In certain preferred embodiments, the structural member comprises a vaso-occlusive platinum coil. The additional vaso-occlusive member may also change shape upon release from the restraining member, for example change from a constrained linear form to a relaxed, three-dimensional configuration upon deployment.

As shown in FIGs. 2 through 5, any of the devices described herein may further comprise a detachment junction (15), which is severable. The detachment junction (15) may be connected to a pusher element, such as a pusher wire (25). The detachment junction can be positioned anywhere on the device, for example at one or both ends of the structural element.

The severable junction(s) may be detached in a variety of ways, for example using an electrolytically detachable assembly adapted to detach by imposition of a current; a mechanically detachable assembly adapted to detach by movement or pressure; a thermally detachable assembly adapted to detach by localized delivery of heat to the junction; a radiation detachable assembly adapted to detach by delivery of electromagnetic radiation to the junction or combinations thereof. Furthermore, the detachment mechanism may be hydraulic, for example the pusher wire may be cannulated, for example to allow for saline injection through the pusher wire to push off the coil.

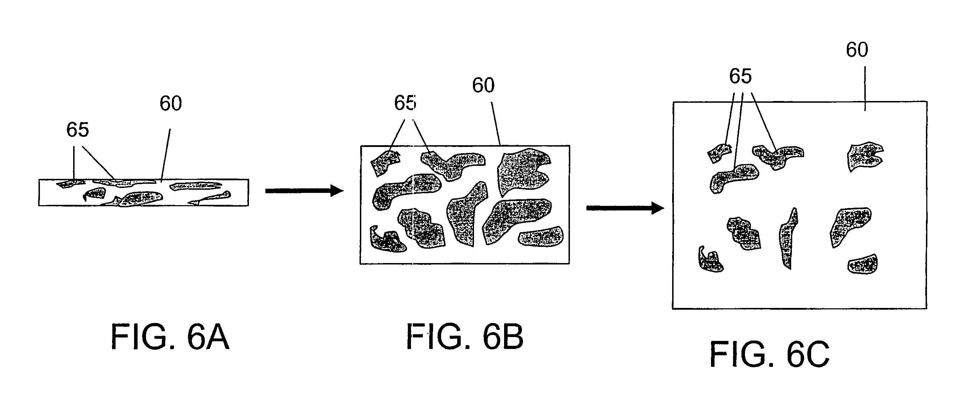

FIGs. 6A-C show an exemplary elastomeric porous material (60) as described herein which also expands to a volume greater than the volume of the uncompressed (native) state. FIG. 6A shows the compressed material (60), including pores (65) in a compressed state. FIG. 6B shows the material (60) in its uncompressed (native) form and FIG. 6C depicts the material (60) in its expanded form. In certain embodiments, the volume of the fully expanded form is at least twice of the volume of the native form.

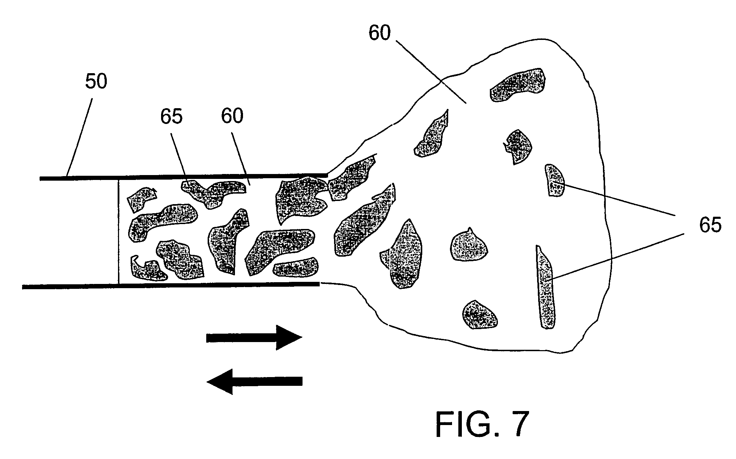

FIG. 7 shows a porous material as described herein partially deployed from a deployment catheter (50). Thick arrows depict how the porous materials described herein are readily deployed from a catheter and readily retracted by compressing the voids, for example to reposition the implant. In other words, the pores are reversibly compressed and expanded, which allows the surgeon greater flexibility in positioning the device. As noted above, it will be apparent that when the porous material is expandable, it will be advantageous to delay expansion relative to implantation so that retrievability is maintained.

The devices described herein may also comprise further additional components, such as co-solvents, plasticizers, coalescing solvents, bioactive agents, antimicrobial agents, porogens, antithrombogenic agents (*e.g*., heparin), antibiotics, pigments, radiopacifiers and/or ion conductors which may be coated using any suitable method or may be incorporated into the element(s) during production. *See, e.g.,* co-owned U.S. Patent Application Publication No. 2005/0149109, U.S. Patent No. 6,585,754 and WO 02/051460, incorporated by reference in their entireties herein. The bioactive materials can be coated onto the device (*e.g*., anticoagulants, growth factors, extracellular matrix components, living cells, DNA fragments, clotting stabilizers, or other materials intended to enhance or encourage wound healing) and/or can be placed in the vessel prior to, concurrently or after placement of one or more devices as described herein.

As noted elsewhere, the location of the device is preferably visible using fluoroscopy. A highly preferred method is to ensure that at least some of the elements (*e.g*., porous component and/or additional vaso-occlusive member) making up the device are provided with significant radio-visibility via the placement of a radio-opaque covering on these elements. A metallic coating of a metal having comparatively more visibility, during fluoroscopic use, than stainless steel is preferred. Such metals are well known but include gold and members of the Platinum Group described above.

One of more of the elements may also be secured to each other at one or more locations. For example, to the extent that various elements are thermoplastic, they may be melted or fused to other elements of the devices. Alternatively, they may be glued or otherwise fastened. Furthermore, the various elements may be secured to each other in one or more locations.

### EXEMPLARY METHODS OF USE

The devices described herein are often introduced into a selected site using the procedure outlined below. This procedure may be used in treating a variety of maladies. For instance in the treatment of an aneurysm, the aneurysm itself will be filled (partially or fully) with the compositions described herein.

Conventional catheter insertion and navigational techniques involving guidewires or flow-directed devices may be used to access the site with a catheter. The mechanism will be such as to be capable of being advanced entirely through the catheter to place vaso-occlusive device at the target site but yet with a sufficient portion of the distal end of the delivery mechanism protruding from the distal end of the catheter to enable detachment of the implantable vaso-occlusive device. For use in peripheral or neural surgeries, the delivery mechanism will normally be about 100-200 cm in length, more normally 130-180 cm in length. The diameter of the delivery mechanism is usually in the range of 0.25 to about 0.90 mm. Briefly, occlusive devices (and/or additional components) described herein are typically loaded into a carrier for introduction into the delivery catheter and introduced to the chosen site using the procedure outlined below. This procedure may be used in treating a variety of maladies. For instance, in treatment of an aneurysm, the aneurysm itself may be filled with the embolics (*e.g*. vaso-occlusive members and/or liquid embolics and bioactive materials) which cause formation of an emboli and, at some later time, is at least partially replaced by neovascularized collagenous material formed around the implanted vaso-occlusive devices.

A selected site is reached through the vascular system using a collection of specifically chosen catheters and/or guide wires. It is clear that should the site be in a remote site, e.g., in the brain, methods of reaching this site are somewhat limited. One widely accepted procedure is found in U.S. Patent No. 4,994,069 to Ritchart, et al. It utilizes a fine endovascular catheter such as is found in U.S. Patent No. 4,739,768, to Engelson. First of all, a large catheter is introduced through an entry site in the vasculature. Typically, this would be through a femoral artery in the groin. Other entry sites sometimes chosen are found in the neck and are in general well known by physicians who practice this type of medicine. Once the introducer is in place, a guiding catheter is then used to provide a safe passageway from the entry site to a region near the site to be treated. For instance, in treating a site in the human brain, a guiding catheter would be chosen which would extend from the entry site at the femoral artery, up through the large arteries extending to the heart, around the heart through the aortic arch, and downstream through one of the arteries extending from the upper side of the aorta. A guidewire and neurovascular catheter such as that described in the Engelson patent are then placed through the guiding catheter. Once the distal end of the catheter is positioned at the site, often by locating its distal end through the use of radiopaque marker material and fluoroscopy, the catheter is cleared. For instance, if a guidewire has been used to position the catheter, it is withdrawn from the catheter and then the assembly, for example including the vaso-occlusive device at the distal end, is advanced through the catheter.

Once the selected site has been reached, the vaso-occlusive device is extruded, for example by loading onto a pusher wire. Preferably, the vaso-occlusive device is loaded onto the pusher wire via a mechanically or electrolytically cleavable junction (*e.g*., a GDC-type junction that can be severed by application of heat, electrolysis, electrodynamic activation or other means). Additionally, the vaso-occlusive device can be designed to include multiple detachment points, as described in co-owned U.S. Patent No. 6,623,493 and 6,533,801 and International Patent publication WO 02/45596. They are held in place by gravity, shape, size, volume, magnetic field or combinations thereof.

It will also be apparent that the operator can remove or reposition (distally or proximally) the device. For instance, the operator may choose to insert a device as described herein, before detachment, move the pusher wire to place the device in the desired location.

Modifications of the vaso-occlusive devices described above in keeping with this invention will be apparent to those having skill in this mechanical and surgical art. These variations are intended to be within the scope of the claims that follow.

## Claims

1. A vaso-occlusive device comprising an elastomeric porous material (10; 60) having a first compressed volume and a second uncompressed volume, wherein
(a) the elastomeric porous material (10; 60) self-expands from the first compressed volume to the second uncompressed volume upon deployment;
(b) the porous material (10; 60) further expands from the second uncompressed volume after deployment to a volume of at least 200% of the said second uncompressed volume; and
(c) the porous material (10; 60), in its fully expanded state, has a nominal pore size greater than about 30 microns and wherein at least 50 percent of the pores (5; 65) are interconnected with an adjacent pore (5; 65).

2. The device of claim 1, wherein the pore size is between about 40 microns and about 400 microns.

3. The device of claim 1 or claim 2, wherein at least 80% of the pores (5; 65) are interconnected with an adjacent pore (5; 65).

4. The device of claim 1, wherein the porous material (10; 60) expands immediately upon deployment.

5. The device of claim 1, wherein the porous, expandable material (10; 60) does not expand immediately upon deployment.

6. The device of any of claims 1 to 5, wherein the porous material (10; 60) comprises a polymer.

7. The device of claim 6, wherein the polymer is selected from the group consisting of silicones, polytetrafluoroethylene, polyesters, polyurethanes, proteins, hydrogel materials and combinations thereof.

8. The device of any of claims 1 to 7, further comprising a radioopaque material.

9. The device of any of claims 1 to 8, further comprising a structural element (20; 40).

10. The device of claim 9, wherein the porous material (10; 60) at least partially surrounds, or is at least partially surrounded by, the structural element (20; 40).

11. The device of claim 10, wherein the porous material (10; 60) at least partially surrounds the structural element (20; 40).

12. The device of claim 10 or claim 11, wherein the structural element (20; 40) at least partially surrounds the porous material (10; 60).

13. The device of claim 9, comprising first and second structural elements (20; 40), wherein the porous material (10; 60) at least partially surrounds the first structural element and the second structural element at least partially surrounds the porous material (10; 60).

14. The device of any of claims 9 to 13, wherein the porous material (10; 60) is attached to the structural element (20; 40) at one or more locations.

15. The device of any of claim 9 to 14, wherein the structural element (20; 40) comprises a metal.

16. The device of claim 15, wherein the metal is selected from the group consisting of nickel, titanium, platinum, gold, tungsten, iridium and alloys or combinations thereof.

17. The device of claim 16, wherein the metal is nitinol or platinum.

18. The device of any of claims 9 to 17, wherein the structural element comprises a coil (20).

19. The device of any of claim 9 to 17, wherein the structural element comprises a coil (20), the coil comprising a stainless steel or super-elastic metal alloy.

20. The device of any of claims 9 to 17, wherein the structural element comprises a tubular braid (40).

21. The device of any of claim 9 to 20, wherein the structural element (20; 40) comprises a biodegradable material.

22. The device of any of claims 9 to 21, wherein the structural element (20; 40) further comprises a detachment junction (15).

23. The device of claim 22, wherein the detachment junction (15) comprises an electrolytically detachable end adapted to detach from a pusher (25) by imposition of a current on the pusher (25).

24. The device of any of claim 1 to 23, further comprising a bioactive component.

## Patentansprüche

1. Eine gefäßverschließende Vorrichtung, umfassend ein elastomeres poröses Material (10; 60), welches ein erstes komprimiertes Volumen und eine zweites nichtkomprimiertes Volumen umfasst, wobei
(a) das elastomere poröse Material (10; 60) von dem ersten komprimierten Volumen zu dem zweiten nichtkomprimierten Volumen nach Einsetzen selbsttätig expandiert;
(b) das poröse Material (10; 60) von dem zweiten nichtkomprimierten Volumen nach Einsetzen zu einem Volumen von mindestens 200% des zweiten nichtkomprimierten Volumen weiter expandiert; und
(c) das poröse Material (10; 60), in seinem vollkommen expandierten Zustand eine nominale Porengröße besitzt, die größer als ungefähr 30 Mikrometer ist und wobei mindestens 50 Prozent der Poren (5; 65) durch eine angrenzende Pore (5; 65) untereinander verbunden sind.

2. Die Vorrichtung gemäß Anspruch 1, wobei die Porengröße zwischen 50 Mikrometern und 400 Mikrometern liegt.

3. Die Vorrichtung gemäß Anspruch 1 oder 2, wobei mindestens 80% der Poren (5; 65) durch eine angrenzende Pore (5; 65) untereinander verbunden sind.

4. Die Vorrichtung gemäß Anspruch 1, wobei das poröse Material (10; 60) sofort nach dem Einsetzen expandiert.

5. Die Vorrichtung gemäß Anspruch 1, wobei das poröse expandierbare Material (10; 60) nicht sofort nach dem Einsetzen expandiert.

6. Die Vorrichtung gemäß einem der Ansprüche 1 bis 5, wobei das poröse Material (10; 60) ein Polymer umfasst.

7. Die Vorrichtung gemäß Anspruch 6, wobei das Polymer aus einer Gruppe bestehend aus Silikonen, Polytetrafluorethylenen, Polyestern, Polyurethanen, Proteinen, Hydrogel-Materialien und Kombinationen dieser ausgewählt ist.

8. Die Vorrichtung gemäß einem der Ansprüche 1 bis 7, weiterhin umfassend ein radioopakes Material.

9. Die Vorrichtung gemäß einem der Ansprüche 1 bis 8, weiterhin umfassend ein strukturelles Element (20; 40).

10. Die Vorrichtung gemäß Anspruch 9, wobei das poröse Material (10; 60) zumindest teilweise das strukturelle Element (20; 60) umgibt oder zumindest teilweise von dem strukturellen Element (20; 60) umgeben wird.

11. Die Vorrichtung gemäß Anspruch 10, wobei das poröse Material (10; 60) zumindest teilweise das strukturelle Element (20; 40) umgibt.

12. Die Vorrichtung gemäß Anspruch 10 oder 11, wobei das strukturelle Element (20; 40) zumindest teilweise das poröse Material (10; 60) umgibt.

13. Die Vorrichtung gemäß Anspruch 9, umfassend erste und zweite strukturelle Elemente (20; 40), wobei das poröse Material (10; 60) zumindest teilweise das erste strukturelle Element umgibt und das zweite strukturelle Element zumindest teilweise das poröse Material (10; 60) umgibt.

14. Die Vorrichtung gemäß einem der Ansprüche 9 bis 13, wobei das poröse Material (10; 60) an einem oder mehreren Orten an dem strukturellen Element (20; 40) angebracht ist.

15. Die Vorrichtung gemäß einem der Ansprüche 9 bis 14, wobei das strukturelle Element (20; 40) ein Metall umfasst.

16. Die Vorrichtung gemäß Anspruch 15, wobei das Metall aus der Gruppe bestehend aus Nickel, Titan, Platin, Gold, Wolfram, Iridium und Legierungen oder Kombinationen derer ausgewählt ist.

17. Die Vorrichtung gemäß Anspruch 16, wobei das Metall Nitinol oder Platin ist.

18. Die Vorrichtung gemäß einem der Ansprüche 9 bis 17, wobei das strukturelle Element eine Spule (20) umfasst.

19. Die Vorrichtung gemäß einem der Ansprüche 9 bis 17, wobei das strukturelle Element eine Spule (20) umfasst und wobei die Spule einen Edelstahl oder eine superelastische Metalllegierung umfasst.

20. Die Vorrichtung gemäß einem der Ansprüche 9 bis 17, wobei das strukturelle Element ein tubuläres Geflecht (40) umfasst.

21. Die Vorrichtung gemäß einem der Ansprüche 9 bis 20, wobei das strukturelle Element (20, 40) weiterhin ein biologisch abbaubares Material umfasst.

22. Die Vorrichtung gemäß einem der Ansprüche 9 bis 21, wobei das strukturelle Element (20; 40) weiterhin eine lösbare Verbindung (15) umfasst.

23. Die Vorrichtung gemäß Anspruch 22, wobei die lösbare Verbindung (15) ein elektrolytisch lösbares Ende umfasst, welches zum Lösen von einem Schieber (25) durch Anlegen eines Stroms an den Schieber (25) ausgebildet ist.

24. Die Vorrichtung gemäß einem der Ansprüche 1 bis 23, weiterhin umfassend einen bioaktiven Bestandteil.

## Revendications

1. Dispositif utilisé pour l'occlusion vasculaire, comprenant une matière poreuse élastomère (10 ; 60) possédant un premier volume à l'état comprimé et un deuxième volume à l'état non comprimé, dans lequel :
(a) la matière poreuse élastomère (10 ; 60) manifeste une expansion automatique à partir du premier volume à l'état comprimé jusqu'au deuxième volume à l'état non comprimé, lors de son déploiement ;
(b) la matière poreuse (10 ; 60) manifeste une expansion ultérieure à partir du deuxième volume à l'état non comprimé après son déploiement jusqu'à atteindre un volume correspondant à au moins 200 % dudit deuxième volume à l'état non comprimé ; et
(c) la matière poreuse (10 ; 60), dans son état complètement expansé, possède une dimension nominale des pores supérieure à environ 30 microns, et dans lequel au moins 50 % des pores (5 ; 65) sont interconnectés à un pore adjacent (5 ; 65).

2. Dispositif selon la revendication 1, dans lequel la dimension des pores se situe entre environ 40 microns et environ 400 microns.

3. Dispositif selon la revendication 1 ou 2, dans lequel au moins 80 % des pores (5 ; 65) sont interconnectés à un pore adjacent (5 ; 65).

4. Dispositif selon la revendication 1, dans lequel la matière poreuse (10 ; 60) subit une expansion directement après son déploiement.

5. Dispositif selon la revendication 1, dans lequel la matière poreuse expansible (10 ; 60) ne subit pas une expansion directement après son déploiement.

6. Dispositif selon l'une quelconque des revendications 1 à 5, dans lequel la matière poreuse (10 ; 60) comprend un polymère.

7. Dispositif selon la revendication 6, dans lequel le polymère est choisi parmi le groupe constitué par des silicones, du polytétrafluoréthylène, des polyesters, des polyuréthanes, des protéines, des matières à base d'hydrogel, ainsi que leurs combinaisons.

8. Dispositif selon l'une quelconque des revendications 1 à 7, comprenant en outre une matière opaque aux radiations,

9. Dispositif selon l'une quelconque des revendications 1 à 8, comprenant en outre un élément de structure (20 ; 40).

10. Dispositif selon la revendication 9, dans lequel la matière poreuse (10 ; 60) entoure au moins en partie l'élément de structure (20 ; 40) ou bien est entourée au moins en partie par celui-ci.

11. Dispositif selon la revendication 10, dans lequel la matière poreuse (10 ; 60) entoure au moins en partie l'élément de structure (20 ; 40).

12. Dispositif selon la revendication 10 ou 11, dans lequel l'élément de structure (20 ; 40) entoure au moins en partie la matière poreuse (10 ; 60).

13. Dispositif selon la revendication 9, comprenant des premier et deuxième éléments de structure (20 ; 40), la matière poreuse (10 ; 60) entourant au moins en partie le premier élément de structure et le deuxième élément de structure entourant au moins en partie la matière poreuse (10 ; 60).

14. Dispositif selon l'une quelconque des revendications 9 à 13, dans lequel la matière poreuse (10 ; 60) est fixée à l'élément de structure (20 ; 40) à un ou plusieurs endroits.

15. Dispositif selon l'une quelconque des revendications 9 à 14, dans lequel l'élément de structure (20 ; 40) comprend un métal.

16. Dispositif selon la revendication 15, dans lequel le métal est choisi parmi le groupe constitué par le nickel, le titane, le platine, l'or, le tungstène, l'iridium, ainsi que leurs alliages ou leurs combinaisons.

17. Dispositif selon la revendication 16, dans lequel le métal est du nitinol ou du platine.

18. Dispositif selon l'une quelconque des revendications 9 à 17, dans lequel l'élément de structure comprend une spirale (20).

19. Dispositif selon l'une quelconque des revendications 9 à 17, dans lequel l'élément de structure comprend une spirale (20), la spirale comprenant un alliage à base d'acier inoxydable ou un alliage à base d'un métal superélastique.

20. Dispositif selon l'une quelconque des revendications 9 à 17, dans lequel l'élément de structure comprend une tresse tubulaire (40).

21. Dispositif selon l'une quelconque des revendications 9 à 20, dans lequel l'élément de structure (20 ; 40) comprend une matière biodégradable.

22. Dispositif selon l'une quelconque des revendications 9 à 21, dans lequel l'élément de structure (20 ; 40) comprend en outre une jonction amovible (15).

23. Dispositif selon la revendication 22, dans lequel la jonction amovible (15) comprend une extrémité apte à se détacher par voie électrolytique, conçue pour se détacher d'un poussoir (25) lorsqu'on applique un courant sur le poussoir (25).

24. Dispositif selon l'une quelconque des revendications 1 à 23, comprenant en outre un composant bioactif.
